# EUROPEAN PATENT SPECIFICATION

(11) **EP 0 680 736 B1**
(45) Date of publication and mention of the grant of the patent: **05.01.2000**
(21) Application number: 95303069.9
(22) Date of filing: 05.05.1995
(51) Int. Cl.: A61F 5/448

(54) **Ostomy coupling**
Ostomiekupplung
Accouplement d'ostomie

(30) Priority: 06.05.1994 GB 9409037
(43) Date of publication of application: 08.11.1995
(73) Proprietor: E.R. Squibb & Sons, Inc., Princeton, N.J. 08540-4000 (US)
(72) Inventor: Steer, Peter Leslie, East Grinstead, Sussex (GB)
(74) Representative: Holmes, Miles Keeton

(56) References cited:
- EP-A- 0 381 393
- EP-A- 0 530 485
- GB-A- 2 109 885
- GB-A- 2 201 345
- US-A- 4 632 436

## Description

This invention relates to an ostomy coupling.

Ostomy couplings are used to connect and disconnect a bag for receiving a stomal discharge to and from a medical grade adhesive pad which is applied to the peristomal area of the skin of the wearer. Many designs of ostomy coupling are known. One which has enjoyed considerable commercial success is described and claimed in GB Patent No. 1,571,657.

It has been proposed by Kubo, in Japanese Utility Model No. 62-11610, published February 1985, that an ostomy device should have a double female ring structure which can interengage with a male ring. The male ring may be on the bag and the female ring on a skin-attachable adhesive pad, or vice-versa. The outer ring on the female ring is circular and flexible and has a pair of inwardly-extending catches at opposite ends of a diameter. By pressing on two diametrically extending lugs, whose diameter is substantially at right angles to the diameter joining the catches, the outer female ring is deformed so that the catches are caused to move radially outwardly, so permitting separation of the two coupling parts.

This arrangement, though perhaps operable in theory, has serious disadvantages in practice, for example (i) to connect or disconnect it is necessary to hold the coupling at four places, approximately spaced at 90° intervals around the periphery, (ii) pressing on two diametrically opposed regions will tend to bend the coupling out of its normal plane and the forces applied may easily cause the body side pad to be partially (or wholly) detached from the skin of the wearer, also the need to press in both ends of the diameter fully, and simultaneously, means that releasing the bagside coupling is subject to uncertainty, (iii) the repeated attachment and withdrawal of the bag side coupling part will cause the o-ring (provided to prevent escape of excreted matter between the male and female rings) to become worn, so compromising its sealing qualities with potentially highly embarrassing and undesirable results, (iv) the wearer may find it difficult to determine whether or not the two coupling parts are properly connected together, and (v) in the case of large sizes, the old and infirm will find it physically difficult to span with their hand and push in diametrally opposed regions of the ring. A further disadvantage of Kubo and of most present day ostomy couplings is that they are not easily manipulated when the bag is to be connected to or disconnected from the pad.

Document GB-A-2201345 discloses an ostomy coupling comprising two coupling parts connectible by a ring, the ring having latch arms which can be flexed between locking and unlocking positions.

It is an aim of this invention to provide an improved design.

According to the present invention, there is provided an ostomy coupling comprising a first coupling element and a second coupling element, the first coupling element being of closed loop form and substantially channel shape as seen in cross-section, and the second coupling element being of complementary closed loop form and having a rib extending upwardly from a flange, the rib carrying a peripheral deflectable seal, the two coupling elements being connectible by a deformable ring which is made captive to one of the first and second coupling elements at one circumferential region thereof the ostomy coupling being characterised in that the deformable ring has a member constrained to move in a radial direction which, when so moved, causes a deformation of the ring whereby tabs thereon are shifted clear of overlap with a rim portion of the other coupling element, so permitting separation of the two coupling elements.

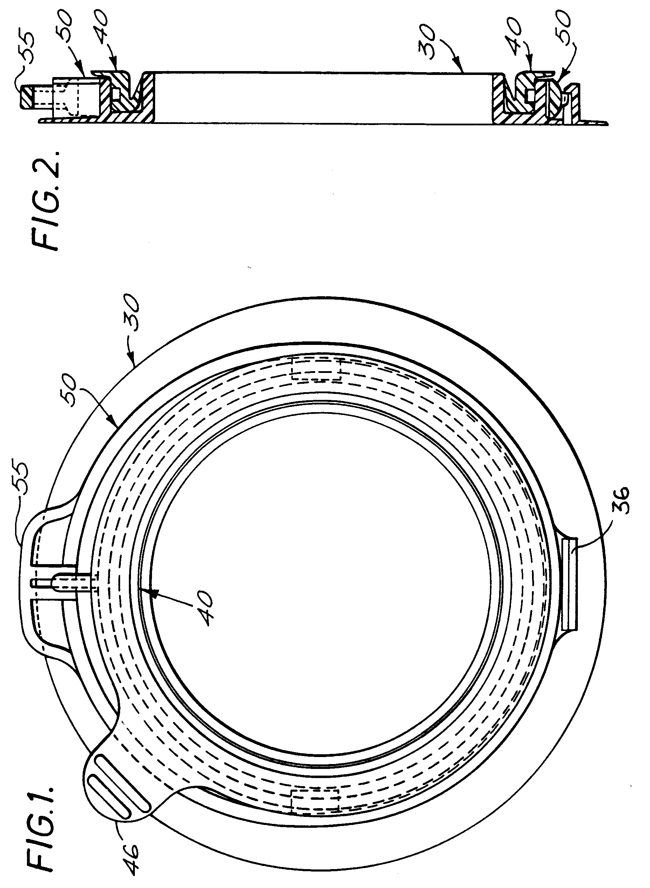

The invention will be better understood from the following description of an illustrative example thereof, given with reference to the accompanying drawings in which:-
Figure 1 is a front view and Figure 2 an axial cross section through one example of ostomy coupling in accordance with the invention;
Figure 3 illustrates a body side coupling element for use in the coupling of Figures 1 and 2;
Figure 4 illustrates a bag side coupling element for use in the coupling of Figures 1 and 2;
Figure 5 illustrates a locking ring, made captive to the body side coupling element, and usable to hold connected or permit detachment of the two coupling elements referred to;

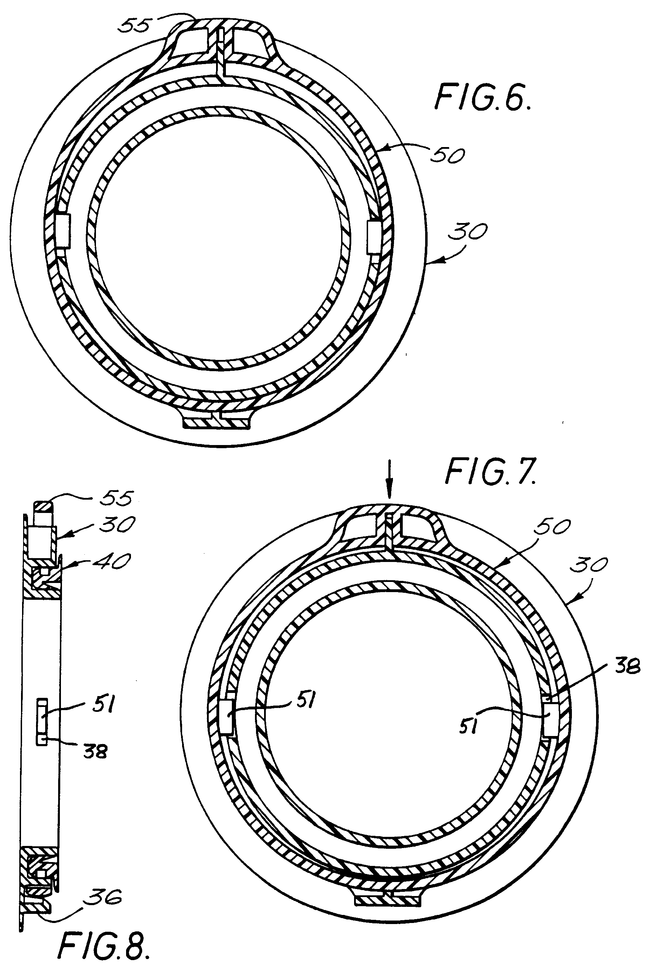

Figure 6 is a front view in schematic form, illustrating the deformable ring in its normal or rest position in which tabs extend radially inwardly to hold the two coupling parts together;
Figure 7 is a view similar to Figure 6, and illustrates the ring in its deformed condition, achieved by a vertical push downwardly (arrow A), and showing the tabs withdrawn so permitting separation of the two coupling parts; and
Figure 8 is a vertical cross-section view showing the position of the parts in the mutually coupled condition.

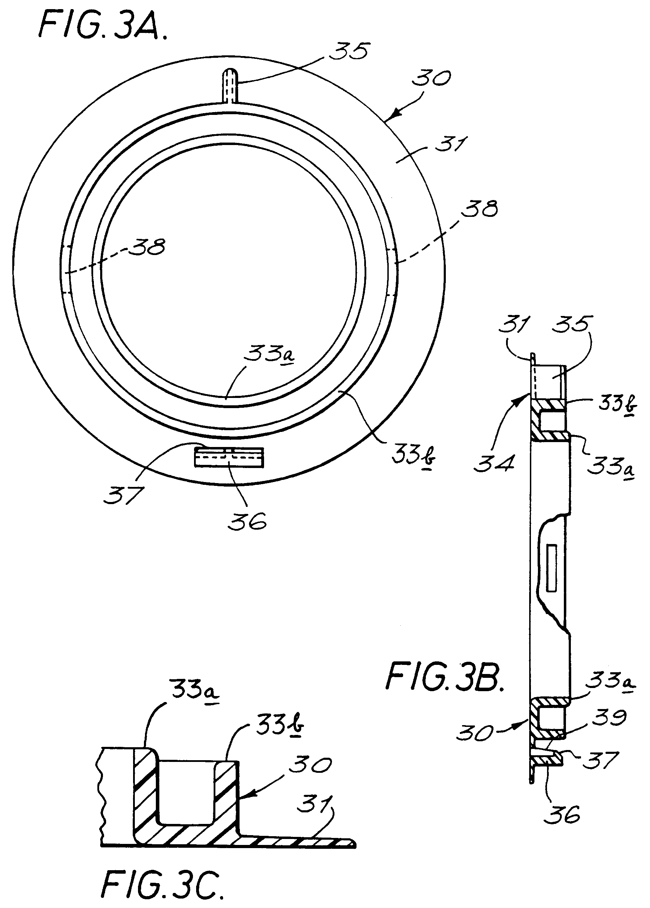

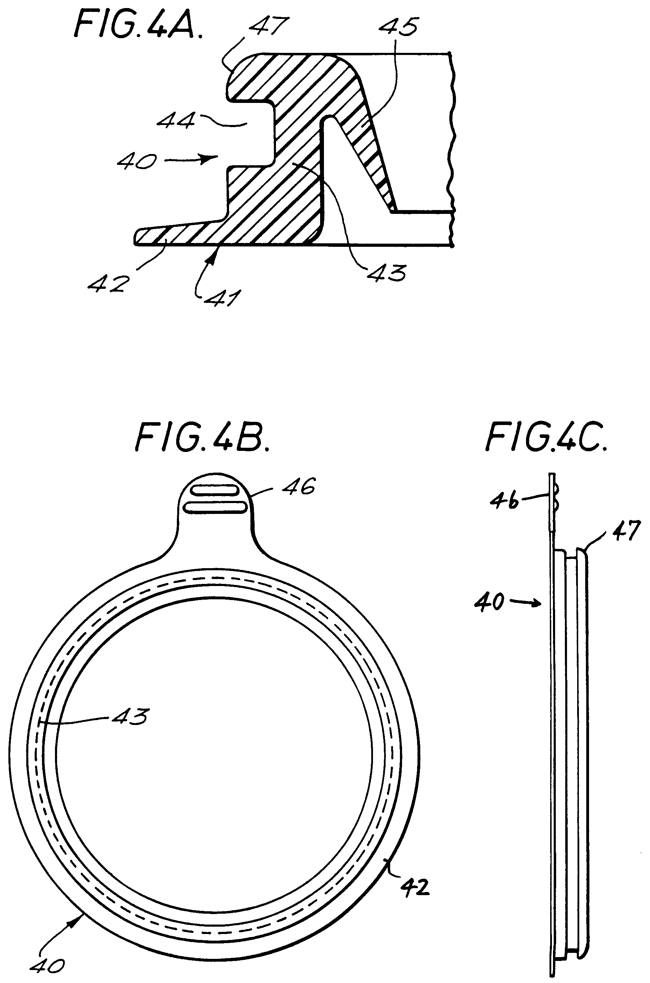

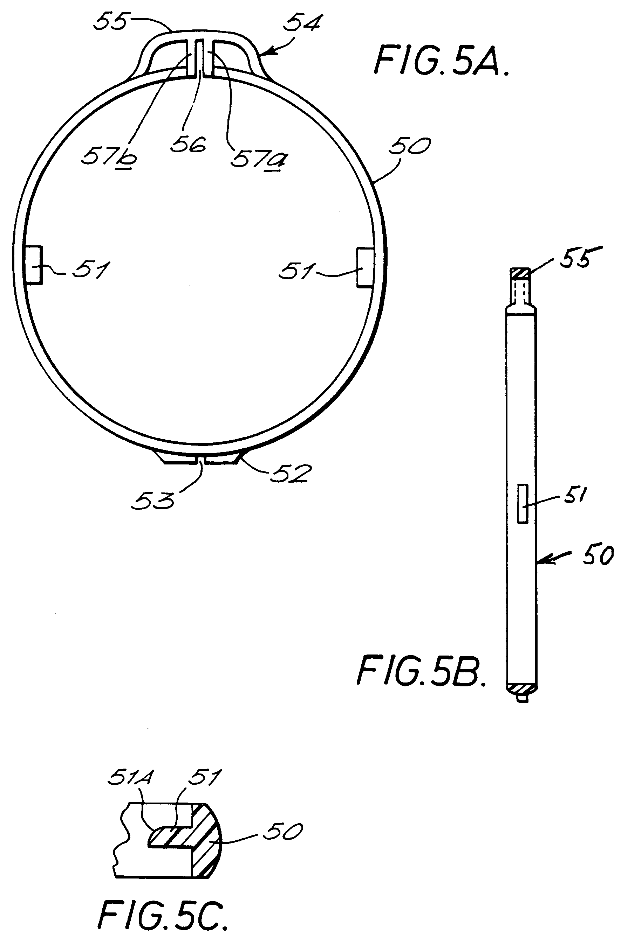

Within Figure 3, Figure 3A illustrates the body side coupling element in front view, Figure 3B in axial section, taken on a vertical line A-A, and Figure 3C is an enlarged cross section showing the channel. Within Figure 4, Figure 4A is a front view of a bag side coupling element, Figure 4B is an enlarged cross sectional view showing the rib, the flange, and the deflectable seal strip of the coupling element of Figure 4A, and Figure 4C is a side view illustrating a peripheral external groove. Referring to Figure 5, Figure 5A is a front view of the preferred form of deformable ring, Figure 5B is an edge view of the said ring, and Figure 5C is a cross section on the portion A-A of Figure 5A, illustrating the tabs which effect an interlock between the body side and bag side coupling elements.

In the preferred embodiment of the invention, the first coupling element is the body side element, and the second coupling element the bag side element. The coupling could be made so that the first coupling element referred to above may be the bag side coupling element and the second coupling element was the body side coupling element. This would be a less-preferred option but might be desirable in certain rare cases. In either case, the manner of connection and dis-connection, using the deformable locking ring, would be the same.

Referring now to Figures 3A-3C, the illustrated body side coupling part 30 has a flange 31 connected to a channel 32 which is U-shaped seen in cross-section. A surface 34 of the coupling 30 has a medical grade adhesive pad, not shown, carried thereon. An upstanding blade or rib 35 is located in an upper region of the coupling and connects to the inner wall 33a of the channel 32. Diametrically opposite to the rib 35, the flange 31 carries a lug 36 which is located radially outside the outer wall 33b of the channel 32. Slots 38 extend through the outer wall at opposite ends of a diameter. The lug 36 has an inwardly directed detent 37, and an inwardly-projecting rib 39. The radially outer wall 33a is slightly higher than the inner wall 33b.

The second coupling part 40 is in this embodiment of the invention the bag side part and it has a surface 41 on a flange 42 to which is attached an ostomy bag. The flange 42 is integral with a rib 43 of closed loop form, preferably cylindrical, and the outer wall of this rib 43 has therein an annular recess 44 whose function will be described shortly. The recess 44 extends 360° around the part 40. Also extending completely around the rib 43, is a flexible deflectable sealing strip 45 whose function is to ensure good sealing between the coupling parts despite manufacturing tolerances and wear induced by coupling and uncoupling of the parts 30 and 40. At one peripheral location, preferably an upper region, a tab 46, Figure 4B, extends from the flange 42. This tab can be gripped by the wearer when it is decided to "peel" the bagside coupling 40 and the bag off the body side coupling 30.

The deformable ring 50 shown in Figure 5 has a pair of inwardly directed tabs 51 diametrically opposite to each other and positioned to be aligned with the slots 38. In its "rest" position it is non-circular (oval) in shape. It also has a flat structure 52 containing a recess 53 which is positioned and dimensioned to receive the rib 39 when the ring 50 is assembled on the coupling part 30. At the opposite end of a diameter to the flat structure 52, the ring 50 has a press-button structure 54, the head of the button being shown at 55. A radially-extending narrow recess 56 is defined by walls 57a and 57b. The recess 56 is positioned and dimensioned to receive the rib 35 on the coupling part 30. The structure of the tabs 51 can be seen from Figure 5C which is a cross section on the line A-A of Figure 5A. The tabs 51 are radiused as shown at 51A; this makes it easy for a wearer, by an axial push, to push the outer wall 39 of the coupling part 30 past the tabs 51 so distorting the ring 50 by elongating its lateral diameter as seen in Figure 5A and reducing it in the direction at 90° to the elongation direction. The ring 50 is made of a springy fairly rigid plastics material (e.g acetal resin, high impact styrene, or various other known engineering plastics materials) and tends to stay in its normal rest configuration (Fig.5A) and to return to that configuration after the deforming is removed.

In operation, the ring 50 is assembled onto the coupling part 30 in the factory, and normally remains on it. In use, a bagside coupling is brought into engagement with the bodyside part by movement in an axial direction and its rib 43 (which is rounded as seen at 47) is pushed past the tabs 52 so temporarily pushing them outwardly and deforming the ring 50. This process is made easier by the radius 51A on each tab. Once the tabs 51 clear the "nose" or rim 47, the resilience of the ring 50 causes them to spring into the recess 44. In this way, the two coupling parts 30 and 40 are securely locked together. During the axial movement described, the sealing strip 45 contacts the radially outer surface of the inner wall 33, and makes good sealing contact with it. In the coupled condition, the flange 42 the outer wall 39.

Advantages of this construction are that withdrawal of the tabs 51 is easily achieved by pushing down the readily accessible push button 55, and that, when coupling, the entry of the tabs 51 into the slots 38 under the automatic restoring force due to the springy nature of the ring gives rise to an audible click. Once the wearer has heard the click, he or she is assured of a sound and secure connection of his/her ostomy bag to the medical pad. This contrasts with the Kubo arrangement, in which a bag could not be fitted to a body-side coupling without first making sure that the Kubo catches are appropriately positioned. Moreover, the resulting combination of adhesive pad and bag according to the preferred embodiment of the present invention, due to the close interfitting and the compact design of the coupling parts, has the advantage of a slim configuration, i.e. small "outstand", which means that the bag is less likely to be visible as a protrusion beneath clothing.

## Claims

1. An ostomy coupling comprising a first coupling element (30) and a second coupling part (40), the first coupling part (30) being of closed loop form and substantially channel shape as seen in cross-section, and the second coupling part (40) being of complementary closed loop form and having a rib (43) extending upwardly from a flange (42), the rib (43) carrying a peripheral deflectable seal (45), the two coupling parts (30, 40) being connectible by a deformable ring (50) which is made captive to one coupling part (30) at one circumferential region (36) thereof the ostomy coupling being characterised in that the deformable ring (50) has a member (54) constrained to move radially inwardly which, when so moved, causes a deformation of the ring (50) whereby tabs (51) thereon are shifted clear of overlap with a rim portion (47) of the other coupling part (40), so permitting separation of the two coupling parts (30, 40).

2. A coupling according to claim 1 in which the ring (50) has a push button structure (54) in its upper region which is constrained to move in a direction radially of the coupling by the engagement of a rib (35) on the first coupling part (30) within an elongated recess (56) in the ring (50).

3. A coupling according to claim 2 in which the tabs (51) on the ring (50) are located at the ends of a diameter which is substantially at right angles to the diameter which intersects the push button structure (54).

4. A coupling according to claim 1, 2 or 3 in which the rib (43) on the second coupling part (40) carries a flexible and deflectible sealing strip (45).

5. A coupling according to any preceding claim including an upstanding structure on the first coupling element (30), located at a lower region thereof, and comprising a radially inwardly extending rib (39) arranged to locate within a recess (53) in the deformable ring (50).

6. A coupling according to any preceding claim in which each of said tabs (51) is radiussed or chamferred at its free end to facilitate fitting of a bagside coupling element (40) to a bodyside coupling element (30) by a substantially axial pushing force.

## Patentansprüche

1. Ostomie-Kupplung mit einem ersten Kupplungselement (30) und einem zweiten Kupplungsteil (40), wobei das erste Kupplungsteil (30) die Form eines geschlossenen Rings und im wesentlichen eine Kanalform im Querschnitt gesehen aufweist, und das zweite Kupplungsteil (40) eine komplementäre Form eines geschlossenen Rings aufweist und einen sich von einem Flansch (42) aus nach oben erstreckenden Steg (43) besitzt, der Steg (43) eine periphere verformbare Dichtung (45) trägt, die zwei Kupplungsteile durch einen verformbaren Ring (50) verbindbar sind, welcher an dem einen Kupplungsteil (30) an dessen Umfangsbereich (36) festgemacht ist, und die Ostomie-Kupplung dadurch gekennzeichnet ist, daß der verformbare Ring (50) ein Element (54) aufweist, das sich erzwungen radial nach Innen bewegt, welches, wenn es so bewegt wird, eine Verformung des Rings (50) bewirkt, wodurch Vorsprünge (51) daran aus einer Überlappung mit einem Randabschnitt (47) des anderen Kupplungsteils (40) verschoben werden und somit eine Trennung der zwei Kupplungsteile (30, 40) ermöglichen.

2. Kupplung nach Anspruch 1, in welcher der Ring (50) eine Drucktastenstruktur (54) in seinem oberen Bereich aufweist, welche auf eine Bewegung in einer Richtung radial zu der Kupplung durch den Eingriff eines Stegs (35) auf das erste Kupplungsteil (30) in einer länglichen Aussparung (56) in dem Ring (50) beschränkt ist.

3. Kupplung nach Anspruch 2, in welcher die Vorsprünge (51) auf dem Ring (50) an den Enden eines Durchmessers angeordnet sind, welcher im wesentlichen im rechten Winkel zu dem Durchmesser angeordnet ist, welcher die Drucktastenstruktur (54) schneidet.

4. Kupplung nach Anspruch 1, 2 oder 3, in welcher der Steg (43) auf dem zweiten Kupplungsteil (40) einen flexiblen und verformbaren Dichtungsstreifen (45) trägt.

5. Kupplung nach einem der vorstehenden Ansprüche, mit einer nach oben ragenden Struktur auf dem ersten Kupplungselement (30), die in dessen unteren Bereich angeordnet ist und einen sich radial nach innen erstreckenden Steg (39) aufweist, der so angeordnet ist, daß er in einer Aussparung (53) in dem verformbaren Ring (50) sitzt.

6. Kupplung nach einem der vorstehenden Ansprüche, in welcher jeder von den Vorsprüngen (51) an seinem freien Ende abgerundet oder abgeschrägt ist, um das Aufsetzen eines beutelseitigen Kupplungselementes (40) auf ein körperseitiges Kupplungselement (30) durch eine wesentliche axiale Drückkraft zu erleichtern.

## Revendications

1. Raccord de stomie comprenant un premier élément de raccord (30) et un second élément de raccord (40), le premier élément de raccord (30) étant en forme de boucle fermée et ayant en coupe sensiblement la forme d'un U, et le second élément de raccord (40) ayant une forme complémentaire de boucle fermée et comportant une nervure (43) qui prolonge une bride (42) vers le haut, la nervure (43) portant un joint d'étanchéité flexible périphérique (45), les deux parties du raccord (30, 40) pouvant être raccordées par une bague déformable (50) qui est prévue captive de l'un des éléments de raccord (30) dans une région circonférentielle (38) de celui-ci, le raccord de stomie étant caractérisé en ce que la bague déformable (50) comporte un élément (54) qui est contraint de se déplacer radialement vers l'intérieur et qui, lorsqu'il se déplace ainsi, provoque une déformation de la bague (50) de sorte que les pattes (51) qu'elle porte cessent de recouvrir partiellement une partie collerette (47) de l'autre élément de raccord (40), permettant ainsi la séparation des deux éléments du raccord (30, 40).

2. Raccord selon la revendication 1, dans lequel la bague (50) comporte dans sa région supérieure une structure de bouton-poussoir (54) qui est contrainte de se déplacer dans une direction radiale du raccord, par engagement d'une nervure (35) du premier élément de raccord (30) à l'intérieur d'un évidement allongé (56) dans la bague (50).

3. Raccord selon la revendication 2, dans lequel les pattes (51) de la bague (50) sont placées aux extrémités d'un diamètre qui forme sensiblement un angle droit avec le diamètre qui coupe la structure de bouton-poussoir (54).

4. Raccord selon la revendication 1, 2 ou 3, dans lequel la nervure (43) du second élément de raccord (40) porte une bande d'étanchéité souple et flexible (45).

5. Raccord selon l'une quelconque des revendications précédentes, comportant une structure verticale sur le premier élément de raccord (30), placée dans une région inférieure de celui-ci, et comprenant une nervure (39) dirigée radialement vers l'intérieur et disposée de façon à se loger à l'intérieur d'un évidement (53) de la bague déformable (50).

6. Raccord selon l'une quelconque des revendications précédentes, dans lequel chacune desdites pattes (51) comporte un rayon ou un chanfrein à son extrémité libre pour faciliter l'adaptation d'un élément de raccord côté poche (40) sur un élément de raccord côté corps (30) au moyen d'une force de poussée sensiblement axiale.
